# EUROPEAN PATENT SPECIFICATION

(11) **EP 0 512 916 B1**
(45) Date of publication and mention of the grant of the patent: **03.02.1999**
(21) Application number: 92401262.8
(22) Date of filing: 06.05.1992
(51) Int. Cl.: A61K 31/557, A61K 9/127

(54) **Liposomal prostaglandin formulations**
Liposomale Prostaglandinformulierungen
Formulation de liposomes contenant des prostaglandines

(30) Priority: 07.05.1991 US 697314; 30.04.1992 US 876200
(43) Date of publication of application: 11.11.1992
(73) Proprietor: THE LIPOSOME COMPANY, INC., Princeton, NJ 08540 (US)
(72) Inventor: Ostro, Marc J., Pennington, NJ 08534 (US)
(74) Representative: VOSSIUS & PARTNER

(56) References cited:
- EP-A- 0 097 481
- EP-A- 0 153 858
- WO-A-88/09170
- STN FILE SERVER: FILE MEDLINE, AN=90080050; J.J. LI et al.: "Impact of C-reactive protein (CRP) on surfactant function", & J. TRAUMA, (1989 DEC) 29(12), 1690-7
- STN FILE SERVER: FILE MEDLINE, AN=89120561; S. OTOMO et al.: "Prostaglandin: E1 incorporated in lipid microspheres (lipo PGE1)", & DRUGS EXP. CLIN. RES., (1985) 11(9), 627-31
- STN FILE SERVER: FILE BIOSIS & BIOL. REV., AN=92-342252; J.A. LEFF et al.: "Post-injury treatment with liposome -encapsulated prostaglandin E-1 decreases acute edematous lung injury ards in rats given interleukin-1 intratracheally", & THIRTY-SECOND ANNUAL MEETING OF THE AMERICAN SOCIETY FOR CLINICAL NUTRITION, Baltimore, Maryland, 30th April - 2nd May 1992. CLIN. RES. 40(2), 1992, 305A
- STN FILE SERVER: FILE BIOSIS & BIOL. REV., AN=92-341390; G. LI et al.: "Attenuation of the no-reflow phenomenon and improved infarct salvage by liposome bound prostaglandin E1 prior to reperfusion", & THIRTY-SECOND ANNUAL MEETING OF THE AMERICAN SOCIETY FOR CLINICAL NUTRITION, Baltimore, Maryland, 30th April - 2nd May 1992. CLIN. RES., 40(2), 1992, 155A
- CHEST, vol. 96, no. 1, July 1989; R.C. BONE et al., pp. 114-119

## Description

### FIELD OF THE INVENTION

This invention relates to liposomal prostaglandin formulations, and more particularly to the use of such formulations in the treatment of cell activation/adhesion syndromes.

### BACKGROUND OF INVENTION

Prostaglandins and related products of eicosatrienoic acid, arachidonic acid and eicosapentaenoic acid metabolism belong to a class of compounds called "eicosanoids", because they are derived from 20-carbon essential fatty acids. See Gilman et al., eds., Goodman and Gilman's The Pharmacological Basis of Therapeutics, Eighth Edition, Pergamon Press, New York, pp. 600-611 (1990), incorporated herein by reference, for a more complete discussion of the structure and synthesis of the eicosanoids, including the prostaglandins, prostacyclins (or prostaglandin I series), thromboxanes and leukotrienes. For purposes of the present discussion, the term "prostaglandins" shall be considered to include the related eicosanoids identified as prostacyclins, thromboxanes, lipoxins and leukotrienes.

As discussed in Gilman et al., supra, the prostaglandins are substances found in essentially all human tissues and body fluids and produce a broad spectrum of effects embracing practically every biological function. They can be considered analogs of a compound with the trivial name prostanoic acid, which structurally comprises a cyclopentane ring and two side chains. Prostaglandins generally fall into several main classes designated by letters and distinguished by substitutions on the cyclopentane ring. Prostaglandins of the E and D series are hydroxy ketones, while the F prostaglandins are diols. The D, E and F series compounds are products of the metabolism of the G and H prostaglandins, and prostaglandins of the A, B and C classes are in turn derived from the corresponding E series compounds. Prostacyclin (PGI) and the thromboxanes are also derived from the G and H series prostaglandins. The main classes are further subdivided in accord with the number of double bonds in the side chains, indicated by subscript 1, 2 or 3. Of particular interest in the present invention is prostaglandin E₁ ("PGE₁").

The prostaglandins have diverse physiological actions such as vasodilative action, improvement of peripheral blood circulation, and antilipolysis. Prostaglandins are known to be therapeutically indicated in many conditions, such as ductus arteriosus, stimulation of uterine contractions leading to induction of labor at term as well as abortion, treatment of bronchial asthma, and suppression of gastric ulceration in animals.

Cell activation and adhesion is a significant problem affecting a wide variety of medical pathologies which shall be referred to herein collectively as "cell activation/adhesion syndromes". Certain cells have been found to exhibit elevated adhesiveness to other cells when adhesion sites on the cell are activated. For example, endothelial cells, such as vascular, plural, pericardial or abdominal endothelial cells, are activated by cytokines such as interleukin-1 (IL-1) or tumor necrosis factor-alpha (TNF-alpha) and/or bacterial endotoxins. In like manner, blood cells, particularly neutrophils and platelets, are activated by agents such as GM-CSF, bacterial endotoxins, bacterial chemoattractants, TNF-alpha, and the like, but especially by the C5a component of complement. Complement is a term for the thermolabile substance in serum that is destructive to certain bacteria and other cells that may be sensitized by a specific complement-fixing antibody. The activated neutrophils and platelets have adhesion sites by which the blood cells stick to each other or adhere to similarly activated endothelial cells. These adhesions result in the formation of clumps and the plugging of small blood vessels, such as those found in the lungs or heart, by adhering to the vascular endothelial cells of the blood vessels, and subsequent degranulation (or release of mediators of cell damage such as superoxide anion (O2-) and proteolytic enzymes). These agents destroy the vascular endothelium and surrounding tissues and also cause capillary damage.

One manifestation of such plugging and degranulation is adult respiratory distress syndrome (ARDS), which is caused by blockages in the small blood vessels of the lungs and subsequent destruction of endothelial cells by activated neutrophils. Another manifestation is rheumatoid vasculitis or systemic vasculitis in which small blood vessels also suffer endothelial lesions. Tissue damage caused by cell activation/adhesion syndrome can also result in increased areas of necrosis in tissue damaged by ischemia or other destructive conditions such as burn injuries or traumatic shock. This is because the damaged tissue releases further amounts of adhesion activating agents, such as C5a, and this further causes additional damage both locally and symmetrically by the cell activation/adhesion syndrome effect, as discussed above. In effect, there is a destructive cycle of tissue damage releasing C5a and other activators, which cause further tissue damage through cell activation/adhesion syndrome and thus the release of more activator.

This cyclic effect is discussed in Abramson et al., "Complement Split Products and the Pathogenesis of SLE," Hospital Practice, 23(12):45-56 (1988), in regard to systemic lupus erythematosus (SLE). In this article it is postulated that intravascular activation of complement triggers release of anaphylatoxins such as C3a and C5a and other complement split products into the circulation. The split products attract and activate inflammatory cells, such as neutrophils and platelets, causing them to aggregate, to adhere to vascular endothelium, to occlude small blood vessels and to release toxic mediators (such as O2-, elastases, and metaloproteases). Activation of circulating complement thus leads to an occlusive vasculopathy, degranulation of neutrophils, and widespread tissue injury.

Thus the cell activation/adhesion syndrome is characterized by the activation of cells by, for example, bacterial endotoxins and C5a. This activation causes the release of toxic mediators which will destroy endothelium and surrounding tissue. Among the cell activation/adhesion syndromes to which the present invention is directed are, among others:
1. Reperfusion injury, such as that related to reperfusion of occluded blood vessels or incidental to transplant surgery or other surgery in which blood flow is temporarily stopped.
2. Septic shock, such as infections which release bacterial endotoxins into circulation.
3. Myocardial infarction,
4. Adult respiratory distress syndrome (ARDS),
5. Rheumatoid vasculitis,
6 Systemic vasculitis,
7. Lupus, which involves circulating immune complexes,
8. Post traumatic shock, and
9. Burn injury.
10. restenosis after angioplasty

The effects of cell activation/adhesion syndrome are further discussed in regard to cardiac reperfusion injury in Seewaldt-Becker et al., "Effect of Anti-Adhesive Antibodies on Reperfusion Injury", in Springer et al., eds., Leukocyte Adhesion Molecules, Springer-Verlag, New York, pp. 138-148 (1990). When there is a blockage in a blood vessel, there is inevitably damage to the endothelial cells of the affected blood vessel, as well as to the ischemic tissue downstream which is denied blood. When the occlusion is cleared, as by physical or chemical means, there is generally further damage to the endothelial cells. Such damaged cells in turn induce activation in neutrophils and platelets. These activated cells then cause activation/adhesion cell damage, which is referred to as "reperfusion injury," after restoration of blood flow.

After reperfusion of an affected blood vessel it is important to restore blood flow as quickly as possible to prevent further tissue damage due to ischemia. However, it has been found that the increased cellular adhesion between neutrophils and endothelial cells inhibits rapid reperfusion in the previously ischemic region, sometimes referred to as the "no-reflow phenomenon". This phenomenon is also affected by the capillary blockage caused by clumping of activated neutrophils and platelets. Furthermore, the adhesion between neutrophils and endothelial cells may damage capillary tissue, allowing various mediators of cell injury released from damaged cells to reach coronary tissue, and thus cause further injury.

The effects of reperfusion injury and other cell activation/adhesion syndromes is also discussed in Springer et al., eds., "Adhesion in Disease and Therapy," Leukocyte Adhesion Molecules, Springer-Verlag, New York, pp. 85-156 (1990).

Prostaglandins, particularly PGE₁ have been found to have potential utility reducing the damage caused by the cycle of cell activation/adhesion syndrome. The same cells which have receptors for activating agents have also been found to have receptors for prostaglandins. It is believed that when prostaglandins, such as PGEs or PGIs, attach to these prostaglandin receptors they deactivate the sites of intercellular adhesion. The mechanism for this is believed to be an increased level within the cells of cyclic AMP via protein kinase A. However, regardless of the mechanism, the key point is that by activation of the prostaglandin receptors, the adhesion sites of the cell are deactivated. Thus, any chemicals capable of activating the prostaglandin sites on cells can break the destructive cycle of the various cell activation/adhesion syndromes. Such a chemical would not even have to be a prostaglandin, but merely a substance which can activate the cell prostaglandin receptors.

The use of prostaglandin to reduce reperfusion injury after the re-opening of occluded blood vessels, such as occurs in the treatment of coronary disease is discussed in Jugdutt et al.. "Dissimilacts of Prostacycn. Prostaglandin E Prostaglandin Eyocardial Infarct ze after Coronarusion in Consciouss. Circulation ch. 49(3):685-700 981). In this article PGE₁ and PGI₂ were found to have a significant effect in reducing the infarct size in dogs which were reperfused after simulation of a myocardial infarction by placement of an occluder snare around a coronary artery. In the reported tests, the prostaglandin was administered via continuous arterial infusion over a six-hour period, resulting in the administration of a relatively large dose of the drug. A reason for this continuous infusion is that prostaglandin such as PGE₁ have an extremely short half life in vivo, and have to be continuously replenished to maintain an effective blood level. Furthermore, it is believed that the prostaglandin is rapidly inactivated when the blood passes through the lungs, which necessitates arterial infusion rather than a simpler intravenous administration. Additionally, the distribution of high levels of PGE₁ in vivo is known to induce systemic effects such as hypotension, tachycardia and diarrhea. In EP-A-0 153 858 the use of a PGE-like prostaglandin for the manufacture of a medicament for the treatment or prevention of a human suffering from or prone to acute respiratory distress syndrome (ARDS) multiple vital organ damage, and for the treatment of shock, trauma, sepsis or any combination thereof is disclosed.

Chest, vol. 96, 1989, pages 114-119, however, reports on a randomized double-blind multicenter study of PGE in patients with ARDS, according to which PGE₁ augmented the hyperdynamic circulation of these patients by reducing systemic and vascular resistance, and in fact decreased the survival rate.

There are problems associated in developing a prostaglandin-based drug. Prostaglandins are very unstable and as such are difficult to produce in a pharmaceutically acceptable formulation. At therapeutic doses, prostaglandins cause hypotension, reflex tachycardia, diarrhea, flusing, somnolence, and abortion in pregnant women: and 95% of the drug is cleared on first pass in the lung liver and kidney (mostly lung) thereby necessitating continuous infusions.

One method proposed for administering prostaglandin in smaller doses is presented in Mizushima et al., "A Multicenter Double Blind Controlled Study of Lipo-PGE₁, PGE₁ Incorporated in Lipid Microspheres, in Peripheral Vascular Disease Secondary to Connective Tissue Disorders." J Rheumatol., 14:97-101 (1987). This article, and the further references cited therein, discusses delivering the drug as "lipo-PGE₁", which is indicated to be in the form of "lipid microspheres", described as being "similar to liposomes". In fact, as described in U.S. Patent No. 4,493,847 issued to Mizushima et al., the "lipo-PGE₁" is a fat emulsion containing the PGE₁ and is not a liposome. However, such emulsions do not offer the stability of the liposome encapsulated PGE₁ used in the present Invention.

Liposomes are completely closed lipid bilayer membranes containing an entrapped aqueous volume. Liposomes may be unilamellar vesicles (possessing a single bilayer membrane) or multilamellar vesicles (onion-like structures characterized by multiple membrane bilayers, each separated from the next by an aqueous layer). The bilayer is composed of two lipid monolayers having a hydrophobic "tail" region and a hydrophilic "head" region. The structure of the membrane bilayer is such that the hydrophobic (nonpolar) "tails" of the lipid monolayers orient toward the center of the bilayer while the hydrophilic "head" orient towards the aqueous phase.

One class of liposomes are those characterized as having substantially equal lamellar solute distribution as set forth in U.S. Patent No. 5,030,453: this class of liposomes is denominated as stable plurilamellar vesicles (SPLV) as defined in U.S. Patent No. 4,522.803 to Lenk et al., monophasic vesicles as described in U.S. Patent No. 4,588,578 to Fountain et al., and frozen and thawed multilamellar vesicles (FATMLV) wherein the vesicles are exposed to at least one freeze and thaw cycle, as described in U.S. Patent No. 4,975,282 to Cullis et al., all incorporated herein by reference.

The lamellarity of a population of liposomes may be reduced and unilamellar vesicles may be produced using an extrusion process such as described in U.S. Patent No. 5,008,050 to Cullis et al., incorporated herein by reference, in which liposomes are extruded under pressure through a filter.

In a liposome-drug delivery system, a bioactive agent such as a drug is entrapped in or associated with the liposome and then administered to the patient to be treated. For example, see Rahman et al., U.S. Patent No. 3,993,754; Sears, U.S. Patent No. 4,145,410; Papahadjopoulos et al., U.S. Patent No. 4,235,871; Schneider, U.S. Patent No. 4,114,179; Lenk et al., U.S. Patent No. 4,522,803; and Fountain et al., U.S. Patent No. 4,588,578.

Lenk et al., U.S. Patent No. 5,082,664 filed May 18, 1988, issued January 21, 1992, incorporated herein by reference, and published as corresponding PCT Application Pub. No. WO 88/09170, December 1, 1988, discloses a method for encapsulating PGE₁, as well as other prostaglandins and eicosanoids, in liposomes.

### SUMMARY OF THE INVENTION

In accordance with one aspect of the present invention the use of a prostaglandin associated with a liposome for manufacturing a pharmaceutical composition is provided for treating a cell activation/adhesion syndrome in an animal, including humans, comprising the step of administering an anti cell activation/adhesion syndrome effective amount of a prostaglandin associated with a liposome. In a particular embodiment of this invention, the prostaglandin is encapsulated in the liposomes.

In a particular embodiment of the present invention, the method is directed to treating a cell activation/adhesion syndrome selected from the group consisting of:
1. Reperfusion injury, such as that related to reperfusion of occluded blood vessels or incidental to transplant surgery or other surgery in which blood flow is temporarily stopped,
2. Septic shock, such as infections which release bacterial endotoxins into circulation,
3. Myocardial infarction,
4. Adult respiratory distress syndrome (ARDS),
5. Rheumatoid vasculitis,
6 Systemic vasculitis,
7. Lupus, which involves circulating immune complexes,
8. Post traumatic shock, and
9. Burn injury.
10. Restenosis after angioplasty

In accordance with the present invention, it has been found that an effective amount of prostaglandin can be delivered in liposomal form to treat a cell activation/adhesion syndrome at a substantially lower dosage rate than when the prostaglandin is administered in free or unbound form. Without being bound by a particular theory or mechanism, it is believed that the liposomes are particularly attracted to the activated cells and adhere to the activated surfaces. The prostaglandin is then readily available at the site of injury to deliver its anti cellular adhesion action.

One theory for the attraction of liposomes to adhesion activated cells is that liposomes are opsonized by fibronectin and vitronectin in the blood. Opsonization is the process by which bacteria are altered such that they become more readily and efficiently engulfed by phagocytes. Thus opsonized liposomes would be more readily attracted to the activated neutrophils which express receptors for fibronectin and vitronectin, thereby delivering the associated prostaglandin to the affected sites.

As discussed above, because of the rapid metabolism of free prostaglandins in vivo, long continuous infusions of relatively large doses of these drugs have been required to maintain an effective blood level in the patient being treated. However, because of the hypotension, tachycardia and diarrhea caused by high blood levels of prostaglandin, it has not been feasible to administer effective amounts of prostaglandins in free form. Furthermore, the high cost of prostaglandins makes it prohibitively expensive to administer such large dosages.

When prostaglandins are administered in the liposomal prostaglandin formulations of the present invention, a much smaller total dosage of the prostaglandin is required to obtain the desired bioactive effect. At the same time, because the prostaglandin is associated with the liposomes, the amount of free prostaglandin in the bloodstream is maintained at a level low enough to prevent or minimize the side effects such as hypotension or diarrhea. Thus, the method of the present invention provides for the effective administration of the prostaglandins at reduced cost and with reduced side effects. In fact, in many treatments, the present invention may provide the only effective way to administer the prostaglandins.

Liposomal-PGE₁ overcomes the problems associated with developing a prostaglandin -based drug. Due to the insertion of the drug into the liposome membrane, followed by lyophilization, the molecule is shielded from water and does not hydrolyze upon long term storage. Liposomal-PGE₁ can remain stable for 1.5 years at refrigerated temperatures and may be stable at room temperature for at least one year. Liposomal PGE₁ has also been effective in the prevention and treatment of ARDS and quite significantly showed no drop in blood pressure, increase in heart rate or diarrhea normally associated with free PGE₁ when given at therapeutic doses. One theory is that as PGE₁ is associated with a liposome its pharmacokinetic behavior is primarily dictated by the liposome and not the drug. Therefore, liposomal-PGE₁ is not cleared from the blood as quickly as free drug and can therefore be given as a quick infusion instead of being infused over several hours or days.

PGE₁ has been shown to be a potent inhibitor of both neutrophil and platelet aggregation, as well as the binding of these cells to activated vascular endothelial cells. It has the ability to both prevent inflammation, that is, prophylaxis, and to turn it off once it has been initiated by any of many factors, that is, treatment. Previously this potential has not been explored due to the aforementioned formulation, toxicologic and pharmacologic problems, which liposomal PGE₁ overcomes.

### DETAILED DESCRIPTION OF THE INVENTION

In accordance with the present invention there is provided the use of a prostaglandin associated with a liposome for manufacturing a pharmaceutical composition for treating a cell activation/adhesion syndrome in an animal, including humans. Such treatment shall be understood to encompass not only the treatment of present symptoms but also the prophylactic administration of such liposomal prostaglandin to prevent the development of such syndromes or to reduce their severity.

In a particular embodiment of the present invention the pharmaceutical composition prepared is used for treating a cell activation/adhesion syndrome selected from the group consisting of:
1. Reperfusion injury, such as that related to reperfusion of occluded blood vessels or incidental to transplant surgery or other surgery in which blood flow is temporarily stopped.
2. Septic shock, such as infections which release bacterial endotoxins into circulation.
3. Myocardial infarction.
4. Adult respiratory distress syndrome (ARDS).
5. Rheumatoid vasculitis.
6 Systemic vasculitis.
7. Lupus, which involves circulating immune complexes.
8. Post traumatic shock.
9. Burn injury. and
10. Restenosis after angioplasty

Tissue destruction as a result of inflammation has been implicated in the pathogenesis of a wide variety of conditions including, among others, reperfusion injury, adult respiratory distress syndrome (ARDS), septic shock, inflammatory bowel disease and rheumatoid arthritis. The inflammatory process involves a complex sequence of events terminated by the release of destructive oxygen species (O2-) and degradative enzymes from neutrophils, white blood cells considered normally to be the body's first line of defense against invading pathogens. In the inflammation process, the normal immune response is activated but the target is host tissue instead of bacteria.

The inflammatory process can be divided into four parts, insult, cell-activation, cell-cell adhesion, and cellular release of mediators of tissue damage. The seminal insult varies from disease to disease. For example, ARDS is induced by factors such as massive trauma or septicemia, cardiac reperfusion injury is caused by the dissolution or dislodging of a clot blocking the coronary artery, restenosis is the result of vascular damage stemming from angioplasty, and septic shock often results from certain types of blood borne infections.

Once an insult occurs, humoral mediators such as interleukin 1 (IL-1), tumor necrosis factor (TNF), peptides produced as a result of complement activation (C5a and C3a, also called anaphlotoxins), and bacterial endotoxins are released into the blood, activating neutrophils and platelets (both white blood cells) and endothelial cells which line the blood vessel walls. All of these cells have specific receptors for each activator. Once binding between the activator and its receptor occurs, the cell switches to a "turned on" or activated state. One of the major changes that occurs on activated cells is the expression of either newly produced receptors or the activation of dormant receptors already present on the cell surfaces. Many such receptors exist, each with a proclivity for a complementary molecule (ligand) on another cell. These receptor-ligand pairs can mediate an adhesion between neutrophils and endothelial cells, neutrophils and neutrophils, platelets and endothelial cells, and platelets and platelets (platelet aggregation).

The last step in the inflammatory process involves the release of O2-and degradative enzymes from either activated neutrophils or platelets (mostly neutrophils) bound to endothelial cells lining the blood vessel walls. The release of these factors can destroy the vascular endothelium allowing the extravasation of inflammatory cells into the surrounding tissue resulting in tissue destruction.

Platelet aggregation, the binding of platelets to each other, can be triggered by many of these same processes. However, instead of the release of O2- or degradative enzymes, platelet aggregation may result in the formation of clots which may block arteries resulting in reocclusion and /or reinfarction.

If the lungs are the main organ that is affected, the condition is referred to as ARDS. If there are other signs present, such as a sharp blood pressure drop, the condition is known as shock. Sepsis/trauma syndrome refers to the broad group of patients suffering from insults such as trauma and infection in whom factors such as TNF, IL-1, etc. are released, with the possibility of shock and organ failure ensuing.

When patients are subject to the insults that can lead to ARDS, such as, to trauma, burns, sepsis, aspiration and hyperoxia, many organs the the body other than the lungs can be affected. The causes and clinical courses of this condition can vary widely. For example, in the case of a patient with a severe infection endotoxin is released from the bacterial cell walls, the inflammatory cascade is initiated, leading to septic shock. Again, as sepsis/trauma syndrome is not limited in causation to infections it is possible that no endotoxin is involved, but nonetheless, the release of factors such as TNF, IL-1 complement and leukotrienes is triggered. The logic and biology involved in the sepsis/trauma syndrome is comparable to that of ARDS, the only difference being that the final target of the cascade involves multiple organs, not just the lungs.

Angioplasty is a technique whereby a balloon is inserted into an occluded artery and inflated in order to open blocked blood vessels. Although this technique has become quite routine in the management of coronary artery disease in the six month period following this procedure, over 33% of the treated patients experience restenosis, or reocclusion of the previously opened blood vessel. It is thought that this condition starts with injury to the vascular endothelium which often results form the balloon procedure. The exposed extracellular matrix will rapidly bind to several layers of activated platelets. Once platelets bind, they will release a variety of growth factors which will result in the proliferation of smooth muscle cells underlying the vessel to the point where the vessel becomes reoccluded. By preventing platelets from binding to the extracellular matrix, one can disrupt the cascade of events resulting in restenosis. Thus, acute administration at the time of the angioplasty procedure of a drug that prevents platelet binding could prevent restenosis.

Recently, De Servi et al., European Heart Journal. "Prostaglandin E administration in unstable angina patients undergoing PTCA; preliminary results", August 1990., published the results of a clinical trial in patients with unstable angina who were given an intracoronary infusion of PGE₁ prior to and following angioplasty. The drug was infused over a 24-hour period. The results of this study showed that the rate of restenosis six months after angioplasty in the PGE₁-treated group was reduced by almost 50% versus the untreated control group, even though the treatment with PGE₁ only lasted 24 hours.

Acute myocardial infarction (more commonly referred to as a heart attack) refers to a blockage of the blood supply to the muscles of the heart, usually caused by a blood clot. If the blood is prevented from reaching the heart for too long, the patient will die. When an occlusion of the coronary artery occurs, the patient is either treated with a fibrinolytic agent, such as tissue plasminogen activator (tPA) or streptokinase, to dissolve the clot, or the blockage may resolve itself. In both instances, blood flow is resumed to the ischemic (oxygen-deprived) region of the heart. This reflow of blood into the heart is called reperfusion. While reperfusion is necessary to save the patient's life, it causes further injury to the heart muscle called reperfusion injury. Reperfusion injury is known to be the end result of the inflammatory cascade.

In addition to the problem of reperfusion injury following clot removal, patients suffering from a myocardial infarction may suffer from other secondary problems. For example, after the normal blood flow is restored to the heart, both neutrophils and platelets are activated. Activated platelets often adhere to one another and begin to reocclude the coronary artery, resulting in a situation where the rate of blood flowing to the heart decreases over time. In some cases, complete reocclusion will occur. PGE₁, in addition to preventing neutrophil binding to endothelial cells, prevents platelet aggregation and reduces, if not eliminates, the no reflow phenomenon.

Sharma et al.,The American Journal of Cardiology, "Intracoronary Prostaglandin E₁ Plus Streptokinase in Acute Myocardial Infarction", page 1161, Dec. 1986, vol. 58, has shown in a clinical setting of acute myocardial infarction that administration of PGE₁ by slow intracoronary infusion together with intracoronary streptokinase provides positive clinical results when compared with a control group taking intracoronary streptokinase alone. The results showed decreased time to reperfusion, reduced dose of streptokinase required, increased percentage of vessels patent after 10 days, and higher ejection fractions. Drawbacks of the study are that the drug must be given by slow intracoronary infusion which is cumbersome and requires specialized facilities and highly trained personnel. Also this approach requires careful titration of the dose of PGE₁ so that significant drops in blood pressure can be seen.

Liposomal PGE₁ is unique in being able to effect both neutrophils and platelets substantially equally.

It has been found that the extracellular release by neutrophils of mediators of inflammation can be modulated by the elevation or depletion of intracellular stores of cyclic adenosine monophosphate (cAMP) and cyclic guanosine monphosphate (cGMP). Elevation of cAMP reduces release of mediators of inflammation whereas increases in the levels of cGMP enhances the excretion of those mediators. cAMP is sometimes referred to as the "universal off-switch" since increasing intracellular levels of cAMP can turn off inflammation, regardless of the factor that initially turned it on. Importantly, some prostaglandins such as PGE₁ elevate cAMP, thus providing the rationale for using PGE₁ as an anti-inflammatory agent. PGE₁ can be thought of as an agent that can activate the "universal switch off'. It has now been shown in vitro that PGE₁ inhibits the binding of neutrophils and platelets to themselves, as well as to endothelial cells, by preventing the activation of the receptors necessary to mediate cell-cell adhesion. This inhibition is coincident with the elevation of intracellular cAMP. Without cell-cell binding, cofactors such as O2- and various degradative enzymes cannot be released, and tissue damage is eliminated. therefore, PGE₁ actually acts as a potent anti-inflammatory agent. It can both prevent inflammation and turn it off, regardless of whether the mediating factor is IL-1, TNF, complement, leukotrienes or others.

The liposomes used in this invention present bioactive eicosanoids, particularly prostaglandins, in association with liposomes. Such association may include the entrapment of the prostaglandin in the liposomes, as well as the association of the prostaglandin with the external or internal membrane surface of the liposomes.

Among the preferred prostaglandins for use in the method of the present invention are the prostaglandins of the E series (PGEs) and the I series (PGIs or prostacyclins), with prostaglandin E₁ showing particular utility in the treatment of cell activation/adhesion syndromes. Such prostaglandins have been found to inactivate the adhesion receptor sites on cells. As discussed above, it is theorized that the prostaglandin activates a prostaglandin receptor site on the cells which in turn causes the deactivation of the adhesion sites. This result may also be achieved by non-prostaglandin compounds which are effective prostaglandin site activators. That is, such compounds may be considered as being "prostaglandin-effective" for purposes of the method of the present invention. For this reason, the term prostaglandin in regard to the method of the present invention is considered to include such prostaglandin-effective compounds.

The "anti-cell activation/adhesion syndrome effective amount" of the prostaglandin associated with a liposome is readily determinable by one skilled in the art. For a particular syndrome, one would conduct dose-ranging trials for the selected liposomal prostaglandin to determine an effective, non-toxic dosage level. For the anti-perfusion injury treatment discussed in Example 3 below, dose-ranging trials were used to determine a suitable dosage was determined to be about 0.5 ug PGE₁ per kg of animal weight. Such dose-ranging may be readily conducted in the same manner for other syndromes.

The mode of administration of the preparation may determine the sites and cells in the organism to which the compound will be delivered. The liposomes of the present invention can be administered alone but will generally be administered in admixture with a pharmaceutical carrier selected with regard to the intended route of administration and standard pharmaceutical practice. The preparations may be injected parenterally, for example, intra-arterially or intravenously. For parenteral administration, they can be used, for example, in the form of a sterile aqueous solution which may contain other solutes, for example, enough salts or glucose to make the solution isotonic. The prostaglandin E₁ liposomes of the present invention, for example, may be given parenterally at a dosage of about 0.5 ug per kg body weight, over a ten-minute period, once or twice a day. Other uses, depending upon the particular properties of the preparation, may be envisioned by those skilled in the art.

The liposomes of the invention may be formed by any of the known methods for forming liposomes, and may be loaded with bioactive agent according to the procedures disclosed in U.S. Patent No. 5,082,664, Lenk et al., as discussed above and incorporated herein by reference. In addition, a similar process for ionizable antineoplastic agents is discussed in Bally et al., PCT Application No. 86/01102, February 27, 1986 and incorporated herein by reference.

In general, any of the methods for forming liposomes mentioned in the Background of the Invention may be used in the practice of the invention, but methods that form unilamellar vesicles are preferred, most preferably large unilamellar vesicles. A preferred method for forming these unilamellar vesicles is the association of bioactive agent (drug, specifically prostaglandin) with the lipid in ethanol similar to the technique of Batzri et al., Biochim. et Biophys. Acta., 298:1015 (1973) using a transmembrane concentration gradient as disclosed in Bally et al. (supra) to load the bioactive agent.

In this technique, lipid and prostaglandin are co-dissolved in an aqueous-miscible organic solvent such as ethanol, then added slowly to a first aqueous solution. Optionally, a preservative such as butylated hydroxytoluene (BHT) may be admixed with the lipid in the solution. The first aqueous solution should include a drying protectant and may additionally include a buffer, such as acetate, citrate or phosphate. The drying protectant may be a saccharide, such as sucrose, maltose, lactose or dextrose, or a combination of saccharides such as either sucrose, maltose, lactose or dextrose with mannitol. Maltose is preferably used. Other saccharides may include, for example, mannose, galactose, raffinose, or trehalose. Alternatively, other protectants such as dexiran, albumin, and poly(vinyl alcohol) maybe used. The buffer may be relatively acidic, that is, having a pH less than about 5.0.

The resulting liposome dispersion may be size-reduced to a more homogenous population, for example, by extrusion through a filter, preferably of 100 nm pore size, the filter being of either the straight path or tortuous path type. A preferred filter for use in this process is an aluminum oxide porous film such as the AnoporeTM filters made by Anotec Separations. These filters are discussed in copending U.S. Patent Application Serial No. 07/593,200, filed October 5, 1990.

Such a population of liposomes may be formed by the extrusion procedures of U.S. Patent No. 5,008,050 to Cullis et al., previously incorporated herein by reference. Such extrusion procedures, wherein the liposomes are passed through a filter under pressure, allow the formation of homogenous populations of liposomes with regard to size. The extrusion may be performed by one or several passes through a filter; for example a straight-through membrane filter (e.g., a NucleporeR polycarbonate filter) or a tortuous path filter (e.g., a NucleporeR MembrafilTM filter (mixed cellulose esters)). When the liposomes are passed more than one time through the filter, the number of passes required will be determined by that necessary to achieve the desired liposome size.

The filter sizes used in the invention are chosen according to the desired size of the final liposome product. In the present invention, liposomes having an average diameter of less than about 200 nm are preferred. The individual liposomes of the present invention are preferably less than about 500 nm in diameter, and are preferably 100 nm to 300 nm in diameter. In the present invention, liposomes of less than about 200 nm diameter are particularly preferred, because liposomes of this size are known to pass through the capillary bed of the lung and are therefore able to pass through to other organs and tissues. Therefore, a filter having a pore size of about 100 nm is chosen for use in the extrusion step.

Other methods for size-reducing the liposomes to a homogenous size distribution as defined hereinabove are ultrasonic exposure, the French Press technique, hydrodynamic shearing, homogenization using for example, a colloid mill or Gaulin homogenizer, or other size reduction techniques.

The resulting size-reduced liposomes are desirably homogenous with regard to size, preferably having a Gaussian size distribution about a mean diameter of less than about 200 nm, with a range of about 20 to about 500 nm. This size-reduced product may be diluted with additional aqueous solution, dried, and stored until use. The size-reduced liposomes may also be sterile filtered, such as by passage through a 220 nm Millipak filter (Millipore, Inc., Bedford, MA).

Immediately prior to use, the lyophilized product may be reconstituted with an aqueous solution having a pH relatively acidic pH, that is, less than about 5.0. Such an adjustment of the pH of the external medium will partition the bioactive agent (prostaglandin) into and/or through the liposome membranes, effectively loading the prostaglandin into the liposomes.

The prostaglandin-associated liposomes may be dehydrated or lyophilized by any method known in the art. This drying procedure requires the addition of a drying protectant to the liposome suspension. This drying protectant prevents the rearrangement of the lipids in the liposome, so that the size and contents are maintained during the drying procedure and through rehydration. Appropriate qualities for such drying protectants are that they be strong hydrogen bond acceptors, and possess stereochemical features that preserve the intramolecular spacing of the liposome bilayer components. It has been found that one group of drying protectant, the saccharide sugars, when included in the liposome formulations, are especially useful at maintaining the liposome particle size after rehydration. This specific group of saccharides comprises, for example, dextrose, sucrose, and maltose, which may be used at about 5 to about 20 percent, preferably at about 10 percent by weight of the aqueous phase. Other saccharides which may be employed are mannose, galactose, raffinose, trehalose, lactose, or triose sugars. Mannitol may be used in conjunction with any of the saccharides, but it has surprisingly been found that when used alone, mannitol does not succeed in maintaining liposome size. Mannitol may be used in concert with the saccharides in about a 0-2% concentration, preferably a 1% concentration. The total concentration of saccharide used ranges from about 5% to about 20%, preferably 10% to 12%, most preferably about 10%. Additional preservatives such as BHT or EDTA in the formulations at, for example, 5 mg BHT per mL of ethanol, and, for example, 0.01% EDTA in 10% dextrose may also be included. Other substances such as urea, albumin, dextran, or poly(vinyl alcohol) may also be used.

The dehydration or lyophilization of the liposomes of the present invention may be performed by any methods known in the art for dehydrating of lyophilizing liposomes. For dehydration, for example, the liposomes may be dried according to the procedures of Janoff et al., U.S. Patent No. 4,880,635, incorporated herein by reference.

Regardless of the dehydration of lyophilization technique, the procedure reduces the aqueous content of a sample to less than about 2%, preferably to less than about 1%. Using the current methods, the liposomal prostaglandin formulation is lyophilized to the point of containing 0-2% aqueous content, and preferably 0-1% aqueous (water) content.

The lyophilized prostaglandin-liposome formulations can be stable for at least one year when stored at 6°C or 25°C. Stability studies using high pressure liquid chromatography (HPLC) analysis of the formulation have shown that after storage at 6°C for one year, no degradation products of the PGE₁ were present.

When the lyophilized liposomes are to be used, rehydration is accomplished by adding an aqueous solution, e.g., distilled water, water for injection (WFI), or buffer or aqueous solution of appropriate pH, as described above, to the liposomes. The liposomes can be then resuspended into the aqueous solution by gentle mixing. The rehydration can be performed at about 25°C.

The following examples are given for purposes of illustration only and not by way of limitation on the scope of the invention.

### EXAMPLE 1

A 1500 mL batch of liposomal PGE₁ was made up of the following components:

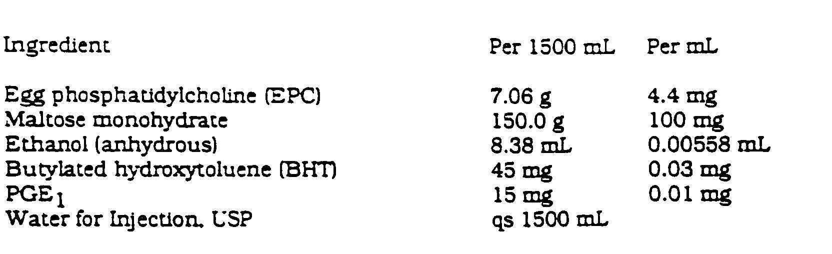

### Preparation:

1350 mL of water for injection was added to a beaker and set with a nitrogen sparge for at least 30 minutes. The 150 g.of maltose (J.T. Baker, Phillipsburg NJ) was added to the water and mixed until dissolved, with the nitrogen sparge continued. This produced 1440 mL of mixture at a pH of 4.81.

In another beaker, the 7.06 g of egg phosphatidylcholine (EPC) (Nippon Oil and Fats, Hyogo, Japan) were combined with 6 mL of ethanol (anhydrous) and mixed until dissolved, and the 45 mg of BHT was added and mixed until dissolved. To this mixture, the 15 mg of PGE₁ was added and mixed until dissolved, the remaining 2.37 mL of ethanol being used to rinse any remaning PGE₁ in the weighing container into the mixture.

The ethanol solution was drawn into a 10 mL capacity glass syringe and injected through a 14 gauge cannula slowly over a period of 11 minutes into the maltose solution with rapid mixing and continued nitrogen sparge. Upon addition of the ethanol/lipid mixture, the solution became cloudy, indicative of the formation of liposomes. The suspension was then diluted to a final volume of 1500 mL with the remaining water for injection.

The liposome dispersion was then extruded 3 times through a 0.2 µm poresize Nuclepore® polycarbonate straight through path type filter (Nuclepore, Pleasanton CA), followed by 5 extrusions through a corresponding 0.1 um filter The particle size of the resulting liposomes was determined to be 0.169 um (S.D. 0.041 um), using quasi-elastic light scattering (QELS) (Nicomp Particle Sizer). The sized liposome dispersion was then passed through a 0.22 µm Millipak sterilization filter. Finally, 10.5 mL aliquots of the dispersion were filled into vials and lyophilized according to the procedures set forth as Example 2 to form a lyophilized product, which can be used immediately or stored for future use.

The lyophilized product was rehydrated with 10 mL of 0.01 M acetate buffer (pH 4.3), with the resultant suspension having pH of about 4.3. Entrapment of the PGE₁ in the liposomes was determined by HPLC, and it was found that at least 98 percent of the available PGE₁ was entrapped in the liposomes, at a concentration of about 9.0 ug PGE₁ per mL of rehydrated suspension.

### EXAMPLE 2

### Lyophilization process

Split-top, butyl-rubber stoppered, Flint lyophilization vials of 50 mL capacity were filled with 10.5 mL of aqueous-suspended liposomes, containing PGE₁. These vials were placed on shelves in the lyophilizer (PV-24 Stokes Lyophilizer). The vials were held at 0°C for 1.5 hours. The shelf temperature was then decreased to -45°C at a rate of 0.8°C per minute, and held for 1 hour, after which a vacuum of 100 µm Hg was applied. The shelf temperature was then increased to -28°C at a rate of 0.5°C per minute, while continuing the vacuum at 100 µg Hg.

The vials were held at -28°C for 50 hours at 100 µm Hg vacuum. The shelf temperature was then increased to +25°C at a rate of 0.5°C/min and held for 22 hours. The vials were then stoppered under partial vacuum.

### Acute Myocardial Infarction

### EXAMPLE 3

### In vivo test of liposomal PGE₁

The rehydrated liposomes of Example 1 were tested in vivo as a means of reducing reperfusion injury incidental to the treatment of myocardial infarction resulting from occluded blood vessels. A total of 53 conditioned 25-35 kg dogs were studied in the dose-ranging trials and the infarction study. Dogs were excluded from analysis for the following reasons: extensive collateralization to the infarct zone (4), heart worms at necropsy (1) and cardiac arrest during occlusion (1). Forty dogs survived of which 27 were suitable for analysis: 7 dogs each in the control, liposomal PGE₁ and liposome-control (empty liposomes) groups and 6 dogs in the PGE₁-control (free PGE₁) group.

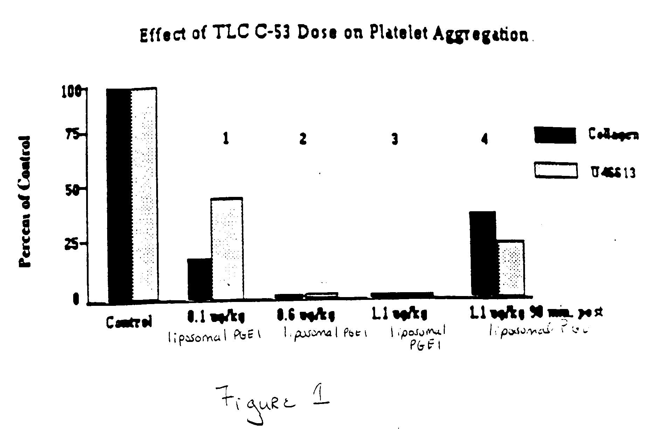

First, dose-ranging trials were conducted to determine a suitable dosage for use in the myocardial infarction studies. Incremental doses of liposomal PGE₁ were given while heart rate, mean arterial pressure, cardiac output and pulmonary capillary wedge pressure were measured. Blood samples were also taken at intervals for platelet aggregation studies (Figure 1). Doses greater than 2.0 µg/kg delivered as a bolus over 10 to 20 minutes produced a marked tachycardia and transient hypotension with a significant increase (doubling) in cardiac output. Platelet aggregation was partially inhibited at 0.1 µg/kg and totally inhibited at 0.6 µg/kg. A dose of 0.5 µg/kg delivered over 10 minutes seemed to increase the heart rate and cardiac output only slightly. In order to optimize the potential results, it was elected to give two doses of liposomal PGE₁ of 0.5 µg/kg each. One injection was administered just after occlusion of the infarct-related artery and the other just prior to reperfusion. This regimen should insure an effect throughout the period of ischemia and initial period of reperfusion. The effect of the liposomal PGE₁ appeared to persist for some time after administration. This observation is significant to reocclusion after reperfusion and restenosis following angioplasty which are directly related to platelet aggregation and adherence to endothelial cells and extracellular matrix.

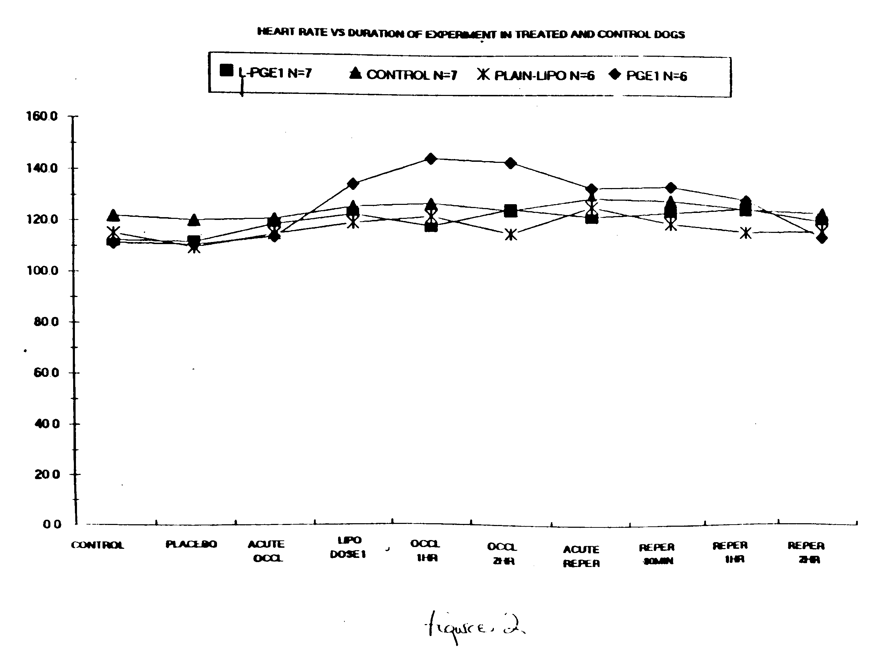

The effects of liposomal PGE₁, free PGE₁, empty liposomes and a control group on heart rate were studied. The test dogs were anesthetized with sodium pentobarbital and maintained with intravenous pentobarbital and InovarR (droperidol/fentanyl). Arterial occlusion was simulated by ligating the left descending aorta artery. Ten minutes after occlusion, 0.5 µg/kg liposome-bound PGE₁ was administered intravenously over ten minutes into a first group of dogs, with a second group of control animals maintained without treatment. The liposome infusion tended to cause an increase in heart rate which was counteracted, in part, by administration of small amounts of additional Inovar. Tests showed no major differences in heart rate throughout the experiment between the control and treated animals. One hundred minutes after occlusion, a second dose of 0.5 µg/kg was administered over ten minutes. After 2 hours, the ligature was removed, causing an acute reperfusion of the blood vessel. Two hours later, the dog was euthanized, and the heart examined for myocardial infarction damage. The results are summarized in Figure 2. The group receiving free PGE₁ exhibited a significant rise in heart rate after the initial dosage and continuing until reperfusion. This rise was not noted in any of the remaining 3 groups of animals. This rise has been attributed to compensatory tachycardia due to vasodilation.

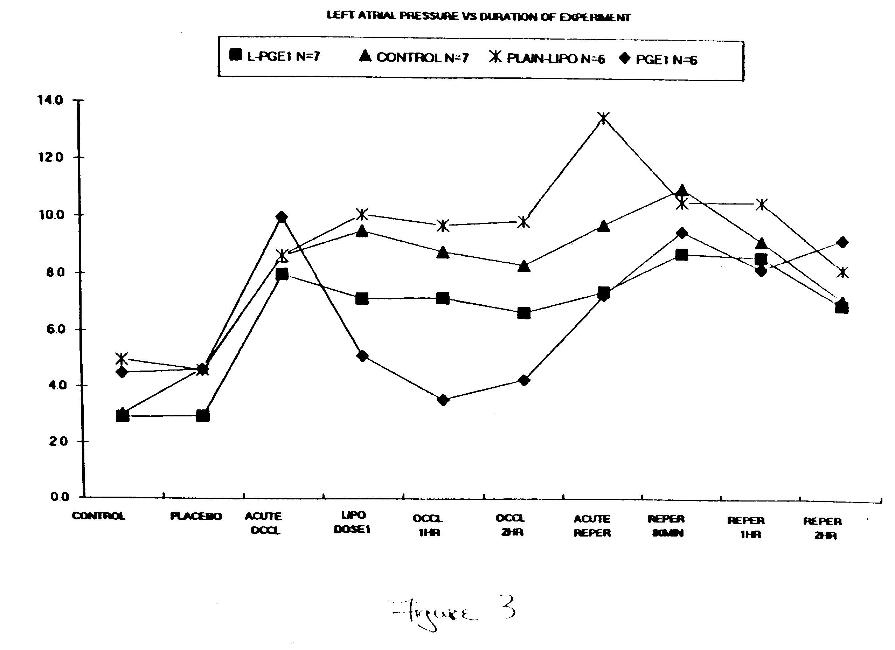

A total of seven dogs were included in the control group, and seven dogs were treated with liposomal PGE₁. During the test period, mean aortic pressure remained relatively constant, and there was no significant difference between the control and treated groups. Mean left arterial pressure increased in each group after occlusion, but there were no significant differences between the groups. Results are summarized in Figure 3.

As anticipated, myocardial blood flow, as measured by 15 um radioactive microspheres, demonstrated a significant decrease in all groups of animals after occlusion. There was no significant improvement in collateral blood flow after liposomal PGE₁ infusion in the test animals suggesting that the microvascular vasodilatory effects of liposomal PGE₁ were minimal.

With reperfusion blood flow increased significantly higher in treated dogs compared to control animals. Examination of the hearts showed that the myocardial infarct size, expressed as a percent of region at risk (i.e. area of low flow during ischemia), appeared reduced by about 50% in the dogs treated with liposomal PGE₁, as compared to the untreated control dogs. In addition, the administration of liposomal PGE₁ resulted in an almost total inhibition of platelet aggregation in the blood.

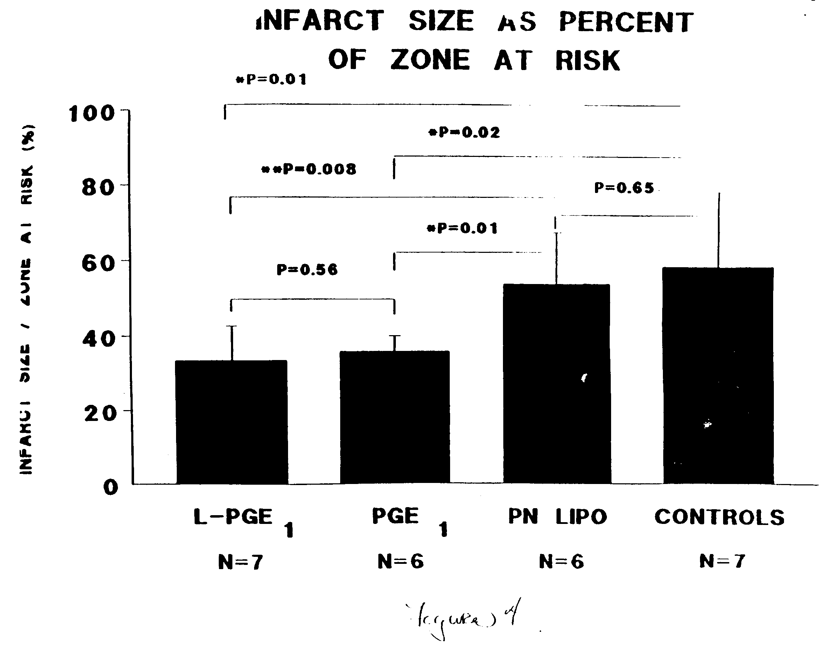

Figure 4 summarizes the results on the four groups of dogs receiving either liposomal PGE₁, free PGE₁, empty liposomes or saline. The liposomal PGE₁ was given in two 0.5 µg/kg infusions over 10 minutes each for a total dosage of 1.0 µg/kg. Free PGE₁ was continusously infused over 90 minutes at 0.1 µg/kg/minute or at a total dosage of 9 µg/kg.

Tests were also conducted to measure the white blood cell infiltration into the ischemic tissue of the heart. Immediately following extraction of the heart from the animals, samples were obtained from the 1) infarct zone, 2) border zone of the infarct zone within the risk region, 3) risk (ischemic) zone, and 4) control zone outside the region at risk. (The region at risk was the portion of the heart supplied by the occluded blood vessel.) The tissue was flash frozen using liquid nitrogen, and kept at -70°C until analyzed. Myeloperoxidase (an enzyme found only in neutrophils) activity was subsequently analyzed for each of the four zones in each animal heart. Intially, six control animals were tested in this manner, although one of these (CONT 4 in the table) was considered aberrant throughout the test, but was included in the tabulation for completeness. Only four of the animals treated with liposomal PGE₁ (LIPO 1-4) were included in this portion of the study. The results are presented in tabular form in Table 1, with the level of myeloperoxidase expressed in arbitrary units of enzyme for comparative purposes:

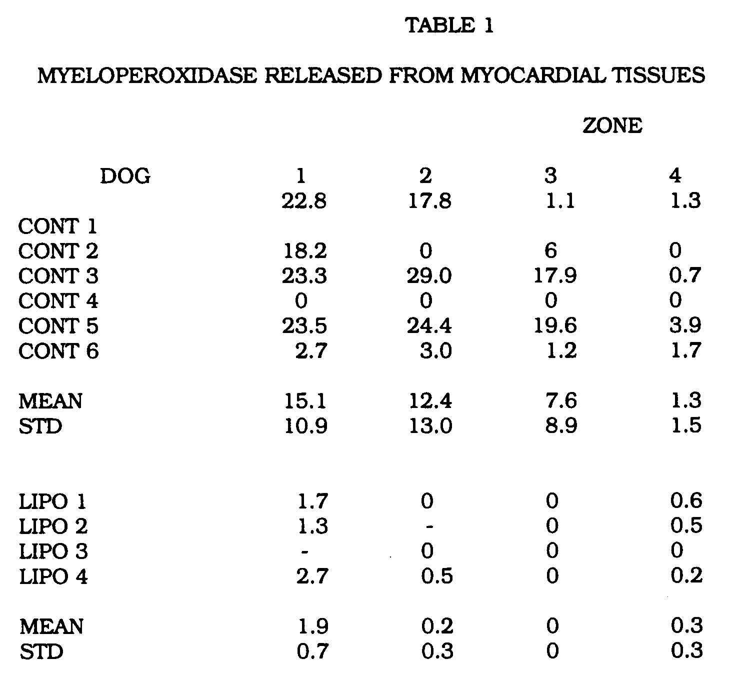

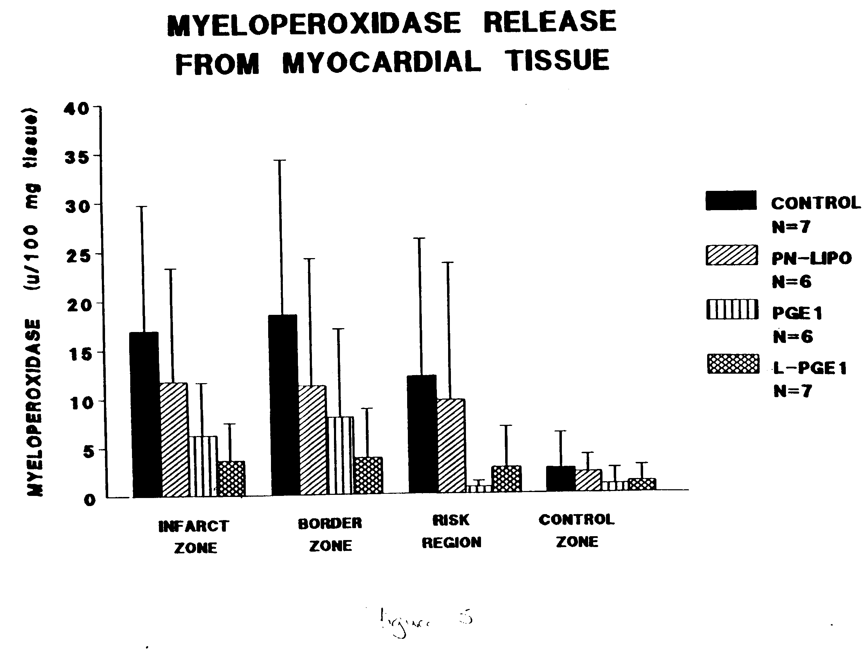

Additional dogs were tested in each group. Figure 5 summarizes the results of the totality of dogs suitable for anaylsis in each of the four groups tested.

In the above table, a dash indicates no measurement was made for that particular value.

### Adult Respiratory Distress Syndrome (ARDS)

ARDS is a condition secondary to a variety of insults including, but not limited to, trauma, burns, sepsis, aspiration and hyperoxia. The condition is characterized by interstitial edema die to capillary injury. Once the lungs fill with fluid, breathing becomes difficult, and 5-60% of the patients die. The sequence of events leading to this catastrophic conclusion can vary depending upon the nature of the initial insult. Factors such as C3a, C5a, IL-1 and TNF activate the neutrophils. The mode of action of PGE₁ is independent of the mechanics of cellular activation. In the presence of PGE₁, neutrophils cannot be activated, and if they are already activated they will be turned off, thereby preventing the disease.

Studies were conducted on rodent models for ARDS where lung injury had been induced by either thermal injury or by direct intratracheal injection of IL-1. As noted in the following examples, doses of liposomal-PGE₁ prevented the leak of fluid into the lung and significantly reduced the influx of neutrophils into the lung.

### EXAMPLE 4

### In vivo tests for prophylaxis of ARDS:

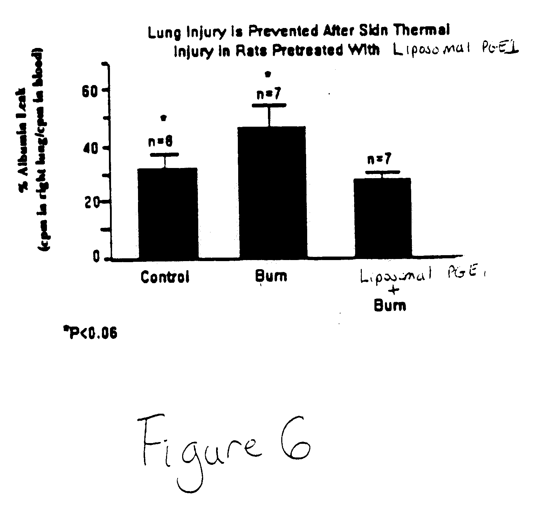

The rehydrated liposomal PGE₁ of Example 1 was tested in vivo as a prophylactic to the onset of adult respiratory distress syndrome (ARDS). Test rats were divided into three groups containing seven or eight animals each and treated with either 8ug/kg of liposomal PGE₁ or saline simultaneous with and one after thermal injury (by immersion in 70°C water for 45 seconds). The first group was treated with liposomal PGE₁, the second group was not treated with the liposomal PGE₁, but was subjected to the same trauma and the third group was neither treated nor traumatized. One hour prior to sacrifice of the rats, 125 I-albumin was injected intravenously as a marker for fluid leak into the lung. Four hours after thermal injury the animals were sacrificed. The results of the study are summarized in Figure 6.

An effect of such traumatization is known to be the onset of ARDS, as evidenced by an increased level of albumin in the lungs of affected animals. The untreated traumatized animals all exhibited elevated levels of albumin in the lungs. Furthermore, six out of these seven test animals died. The level of albumin in the animals treated with liposomal PGE₁ was found to be about as low, or even lower, than the levels in the untraumatized control group. Furthermore, none of the seven PGE₁ treated animals died as a result of the trauma. These results show the effectiveness of liposomal PGE₁ as a prophylactic to the onset of ARDS.

### EXAMPLE 5

In vivo tests for treatment of ARDS induced by intratracheal injection of IL-1 :

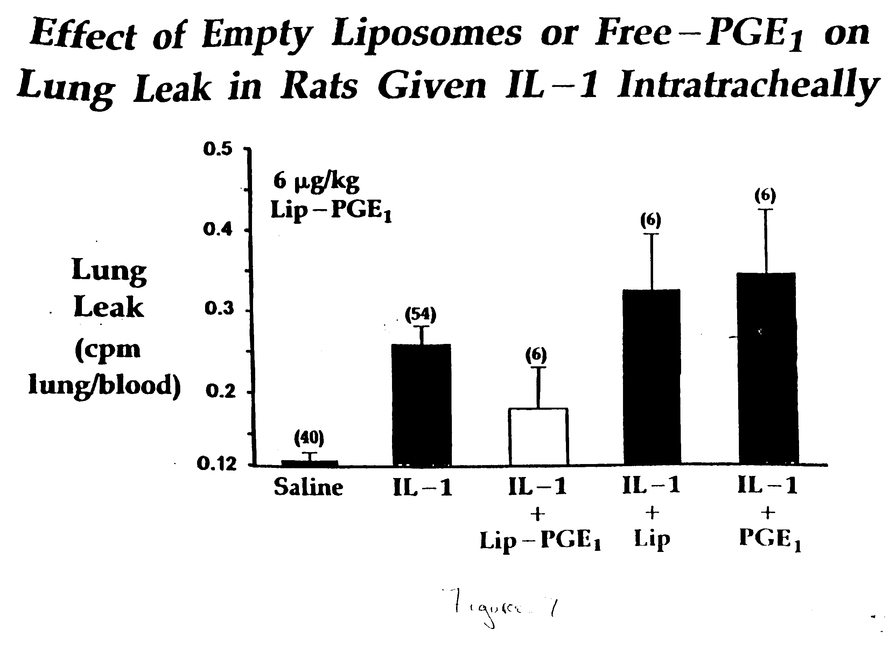

Intratracheal instillation of recombinant IL-1 induces a neutrophil influx and lung injury in rats (Figure 7). Liposomal PGE₁ treatment will decrease IL-1 induced lung injury and neutrophil influx. 50 ng of commercially available interleukin 1 (IL-1) or saline (in the control group) was instilled into the trachea of rats inducing neutrophil infiltration into the lungs and leak of fluid into the alveoli. Intravenous treatment, 6 µg/kg, of either rehydrated liposomal PGE₁ of Example 1, free PGE₁ or empty liposomes was administered 2.5 hours after IL-1 instillation in rats having received the IL-1. Of the rats analyized 40 received only saline, 54 received IL-1 and were untreated, 6 received IL-1 and were treated with liposomal PGE₁, 6 received IL-1 and were treated with empty liposomes, and 6 received IL-1 and were treated with free PGE₁.

Four and a half hours after the IL-1 was given, after many neutrophils had already infiltrated into the tissue, the rats were injected with 125 I albumin as a marker into the bloodstream. A half an hour after the albumin was injected the animals were sacrificed, the lung removed and the leak of fluid into the lung was quantitated by determining the amount of isotope in the tissue. A lung leak index was calculated based on the count per minute (cpm) of labeled 125 I-albumin in the lung divided by the amount of labeled 125 I-albumin in the bloodstream. The lung leak is expressed on the Y-axis of Figure 7. As noted in Figure 7, instillation of IL-1 without treatment caused a 2.5-fold increase of lung leak above sham or animals given saline. However, when liposomal PGE₁ treatment was given after instillation of IL-1, the lung leak index was equal to that seen in the sham control. Importantly, treatment with neither the free PGE₁ nor the empty liposomes had any effect on lung leak.

This sequence of events represents that which might occur in a clinical setting. Injury was induced, and treatment was initiated at a later time after the appropriate diagnosis was made.

The terms and expressions which have been employed herein are used as terms of description and not of limitation, and there is no intention in the use of such terms and expression of excluding any equivalents of the features described or portions thereof, but it is recognized that various modifications are possible within the scope of the invention claimed.

## Claims

1. Use of a prostaglandin associated with a liposome for manufacturing a pharmaceutical composition for treating a cell activation/adhesion syndrome in mammal.

2. Use of claim 1, wherein the prostaglandin is associated by being encapsulated in said liposome.

3. The use of claim 1 or 2, wherein the mammal is human.

4. The use of anyone of claims 1 to 3, wherein the cell activation/adhesion syndrome is selected from the group consisting of reperfusion injury, septic shock, myocardial infarction, adult respiratory distress syndrome, rheumatoid vasculitis, lupus, post traumatic shock, burn injury and restenosis after angioplasty.

5. The use of claim 4, wherein the cell activation/adhesion syndrome is reperfusion injury.

6. The use of claim 4, wherein the cell activation/adhesion syndrome is adult respiratory distress syndrome.

7. The use of anyone of claims 1 to 6, wherein said prostaglandin is a prostaglandin E or a prostaglandin I.

8. The use of claim 7, wherein said prostaglandin is prostaglandin EI.

## Patentansprüche

1. Verwendung eines Liposomen-assoziierten Prostaglandins zur Herstellung eines Arzneimittels für die Behandlung eines Zellaktivierungs-/Zelladhäsionssyndroms in einem Säuger.

2. Verwendung nach Anspruch 1, wobei das Prostaglandin dadurch assoziiert ist, daß es in dem Liposom eingekapselt ist.

3. Verwendung nach Anspruch 1 oder 2, wobei der Säuger ein Mensch ist.

4. Verwendung nach einem der Ansprüche 1 bis 3, wobei das Zellaktivierungs-/Zelladhäsionssyndrom eine Reperfusionsverletzung, septischer Schock, Herzinfarkt, akute respiratorische Insuffizienz (ARDS), rheumatoide Vasculitis, Lupus, posttraumatischer Schock, eine Verbrennung oder eine Restenose nach einer Angioplastie ist.

5. Verwendung nach Anspruch 4, wobei das Zellaktivierungs-/Zelladhäsionssyndrom eine Reperfusionsverletzung ist.

6. Verwendung nach Anspruch 4, wobei das Zellaktivierungs-/Zelladhäsionssyndrom eine akute respiratorische Insuffizienz (ARDS) ist.

7. Verwendung nach einem der Ansprüche 1 bis 6, wobei das Prostaglandin ein Prostaglandin E oder ein Prostaglandin I ist.

8. Verwendung nach Anspruch 7, wobei das Prostaglandin Prostaglandin EI ist.

## Revendications

1. Utilisation d'une prostaglandine associée à un liposome pour fabriquer une composition pharmaceutique destinée au traitement d'un syndrome d'activation/adhésion cellulaire chez un mammifère.

2. Utilisation selon la revendication 1, dans laquelle la prostaglandine est associée en étant encapsulée dans ledit liposome.

3. Utilisation selon la revendication 1 ou 2, dans laquelle le mammifère est un sujet humain.

4. Utilisation selon l'une quelconque des revendications 1 à 3, dans laquelle le syndrome d'activation/adhésion cellulaire est choisi dans le groupe constitué de la lésion par reperfusion, du choc septique, de l'infarctus du myocarde, du syndrome de détresse respiratoire chez l'adulte, de la vascularite rhumatoïde, du lupus, du choc post-traumatique, des lésions par brûlures et de la resténose après angioplasie.

5. Utilisation selon la revendication 4, dans laquelle le syndrome d'activation/adhésion cellulaire est une lésion par reperfusion.

6. Utilisation selon la revendication 4, dans laquelle le syndrome d'activation/adhésion cellulaire est le syndrome de détresse respiratoire chez l'adulte.

7. Utilisation selon l'une quelconque des revendications 1 à 6, dans laquelle ladite prostaglandine est une prostaglandine E ou une prostaglandine 1.

8. Utilisation selon la revendication 7, dans laquelle ladite prostaglandine est la prostaglandine E1.
